# EUROPEAN PATENT APPLICATION

(11) **EP 3 741 297 A1**
(43) Date of publication of application: **25.11.2020**
(21) Application number: 19739051.1
(22) Date of filing: 15.01.2019
(51) Int. Cl.: A61B 5/0408

(54) **BIOELECTRODE**

(30) Priority: 15.01.2018 JP 2018004456
(71) Applicant: NOK Corporation, Minato-ku Tokyo 105-8585 (JP)
(72) Inventor: FUTASHIMA Ryo, Tsujido-shinmachi, Fujisawa-shi, Kanagawa 251- 0042 (JP); SUGIYAMA Yasushi, Tsujido-shinmachi, Fujisawa-shi, Kanagawa 251- 0042 (JP); UDA Toru, Tsujido-shinmachi, Fujisawa-shi, Kanagawa 251- 0042 (JP)
(74) Representative: TBK
(86) International application number: PCT/JP2019/000912
(87) International publication number: WO 2019/139165

(57) **Abstract**

Provided is a bioelectrode having excellent electrical conductivity.

A bioelectrode (1) includes a conductive rubber electrode (11) containing a rubber and conductive carbon particles, and a silver coating layer (12) provided on the conductive rubber electrode (11) and containing a silicone rubber and silver particles, wherein the silver coating layer (12) further contains a water absorbent polymer formed of a modified polyalkylene oxide.

## Description

### Technical Field

The present invention relates to a bioelectrode, and more specifically relates to a bioelectrode having excellent electrical conductivity.

### Background Art

Bioelectrode materials made of metal such as sheets of highly conductive metals, for example, gold, silver, platinum, and copper have been conventionally used in bioelectrodes. These bioelectrode materials made of metal have poor adhesion to the skin, and detection of electrical signals from the skin is insufficient. When the materials are used as bioelectrodes, it is necessary to apply a gel, cream, paste, or the like to the skin. Additionally, metals, which are rigid, are not suitable to adhere for a long period.

There also exist bioelectrodes composed of an adhesive material such as a gel (also referred to as gel electrodes) (e.g., see Non-Patent Literature 1). In these bioelectrodes, application of a gel, cream, paste, or the like is not required, but trash and dust are likely to adhere to the adhesive material, and the adherence is gradually lost. For this reason, such bioelectrodes are not suitable for repetitive use. There are also known electrodes obtained by compounding carbon nanotubes in a rubber (e.g., see Patent Literature 1).

### Document List

### Patent Literature

Patent Literature 1: Japanese Patent Application Publication No. 2015-41419

### Non-Patent Literature

Non-Patent Literature 1: The Japanese journal of medical instrumentation, Vol. 80, No. 1 (2010) P28 - P37

### Summary of Invention

### Technical Problem

Electrodes in which carbon nanotubes and a conductive filler such as a metal powder of Cu, Ag, Au, Al, Ni, and the like are compounded exhibit electrical conductivity by direct contact among filler constituents in the rubber. However, further room for improvement has been found in the electrical conductivity.

It is an object of the present invention to provide a bioelectrode having excellent electrical conductivity.

### Solution to Problem

A bioelectrode according to the present embodiment is characterized by comprising: a conductive rubber electrode containing a rubber and conductive carbon particles; and a silver coating layer provided on the conductive rubber electrode and containing a silicone rubber and silver particles, wherein the silver coating layer further contains a water absorbent polymer formed of a modified polyalkylene oxide.

In the bioelectrode according to the present embodiment, the silver coating layer preferably includes ions of ion conduction present among the silver particles.

In the bioelectrode according to the present embodiment, the ions preferably include at least one selected from the group consisting of chloride salts, sulfates, and carbonates.

In the bioelectrode according to the present embodiment, the melting point of the water absorbent polymer is preferably equal to or lower than the crosslinking temperature of the silicone rubber.

In the bioelectrode according to the present embodiment, the silver coating layer preferably further contains a modified silicone.

In the bioelectrode according to the present embodiment, the modified silicone preferably contains at least one selected from the group consisting of polyether-modified silicones, polyether-alkyl-comodified silicones, polyglycerin-modified silicones, and polyglycerin-alkyl-comodified silicones.

### Effects of Invention

According to the present invention, it is possible to provide a bioelectrode having excellent electrical conductivity.

### Brief Description of Drawings

[Fig. 1] A cross-sectional view conceptually illustrating one example of a bioelectrode according to an embodiment of the present invention.
[Fig. 2] A view conceptually illustrating one example of ion conduction facilitation by an electrical conductivity improving material (modified silicone) according to the embodiment of the present invention.
[Fig. 3] A view conceptually illustrating a usage example of the bioelectrode according to the embodiment of the present invention.
[Fig. 4] A view illustrating electrocardiogram waveforms of an adult male measured using the bioelectrode according to the embodiment of the present invention.
[Fig. 5] A conceptual view of a conveyor belt used in a bending test.
[Fig. 6] A view illustrating dependency of the surface resistance on the number of times of bending.

### Description of Embodiments

Hereinafter, embodiments of the present invention will be described with reference to the accompanying drawings. Note that the present invention is not limited by the following embodiments in any way.

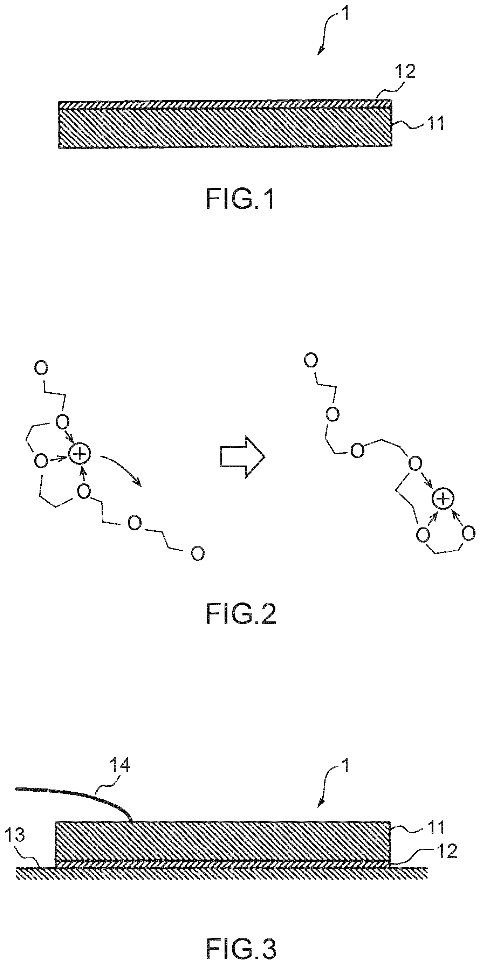

Fig. 1 is a cross-sectional view conceptually illustrating one example of a bioelectrode 1 according to a present embodiment. The bioelectrode 1 according to the present embodiment includes a silver coating layer 12 on a conductive rubber electrode 11.

The conductive rubber electrode 11 is obtained by compounding conductive carbon particles in a rubber. The conductive rubber electrode 11 forms a main body of the bioelectrode, and the entire shape of the bioelectrode is imparted by the shape of the conductive rubber electrode 11.

As the rubber constituting the conductive rubber electrode 11, for example, a silicone rubber and the like can be preferably used. The silicone rubber is not especially limited, but preferably used are organosilicon polymers and the like having a siloxane bond (-Si-O-) as the main chain and having a group such as a methyl group, a phenyl group, or a vinyl group or hydrogen as a side chain.

The conductive carbon particles are not limited as long as the particles can impart electrical conductivity to a rubber such as the silicone rubber mentioned above. Examples of the conductive carbon particles include carbon black and graphite. Examples of carbon black include Ketjen black and acetylene black. Among these, as the carbon black, Ketjen black and the like are preferable from the viewpoint of relatively high electrical conductivity.

The average particle size of the conductive carbon particles is not especially limited, but is preferably in the range of 0.1 µm or more and 100 µm or less, more preferably in the range of 1 µm or more and 30 µm or less. The average particle size is an average diameter determined by measurement of an electron micrograph and calculation using arithmetic mean.

The amount of the conductive carbon particles to be compounded in the conductive rubber electrode 11 is not especially limited. The amount can be appropriately set in the range where electrical conductivity can be imparted, and is preferably in the range of 10% by mass or more and 70% by mass or less, more preferably in the range of 20% by mass or more and 50% by mass or less.

The silver coating layer 12 is obtained by compounding silver particles to a silicone rubber. The silicone rubber is not especially limited, but preferably used are organosilicon polymers and the like having a siloxane bond (-Si-O-) as the main chain and having a group such as a methyl group, a phenyl group, or a vinyl group or hydrogen as a side chain.

When the silver coating layer 12 is constituted with a silicone rubber, the silicone rubber serves as a binder for the silver particles. Especially, the silver coating layer 12 is retained on the conductive rubber electrode 11 formed of the silicone rubber with a high adhesion, and thus it is possible to prevent the layer 12 from coming off from the electrode 11. This adhesion also contributes to stabilization of electrical connection between the silver coating layer 12 and the conductive rubber electrode 11. Additionally, a silicone rubber has excellent flexibility, and thus, conformability to movements of a living body is suitably exhibited during use of the bioelectrode. As a result of these, it is possible to suitably reduce the contact impedance with a living body.

In the present embodiment, the silver coating layer 12 contains a water absorbent polymer formed of a modified polyalkylene oxide.

The silver particles are dispersed, by the water absorbent polymer formed of a modified polyalkylene oxide, in a state optimal for forming a network of the conductive particles (silver particles) in the silver coating layer 12. This can reduce the surface resistance of the bioelectrode (i.e., the surface resistance of the silver coating layer 12) and enhance the electrical conductivity.

Particularly, the water absorbent polymer formed of a modified polyalkylene oxide has a melting point equal to or lower than the crosslinking temperature of the silicone rubber (curing treatment temperature). Thus, the water absorbent polymer melts on crosslinking of the silicone rubber, exhibits high affinity to both the silver particles and the binder (silicone rubber), allows the silver particles and the binder to be mixed well, and can facilitate construction of a network among the silver particles. Considering that the crosslinking temperature (curing treatment temperature) of an ordinary silicone rubber is about 150°C to 200°C, the melting point of the water absorbent polymer formed of a modified polyalkylene oxide is preferably 120°C or less, more preferably 100°C or less.

As the water absorbent polymer formed of a modified polyalkylene oxide, it is possible to use a polyalkylene oxide imparted with a crosslinking structure so as to be modified to function as a water absorbent polymer, for example. Specific examples include reaction products of reaction (polymerization) between polyethylene oxide and a polymerizable monomer. Examples of the polymerizable monomer include urethane-based monomers such as 1,4-butanediol and dicyclohexylmethane-4,4'-diisocyanate. As the modified polyalkylene oxide, nonionic-type ones can be preferably used. Such modified polyalkylene oxides are available as commercially available products, and an example thereof is trade name: "AQUA CALK TWB"; pulverized product, melting point: 60 to 65°C (manufactured by Sumitomo Seika Chemicals Company, Limited).

In the present embodiment, the silver coating layer 12 preferably includes ions for ion conduction present among the silver particles. Accordingly, the silver coating layer 12 can have electron conductivity derived from the silver particles and ion conductivity derived from ions present among the silver particles. Herein, "electron conduction (conductivity)" means that electrons singly (not as ions) migrate to thereby exhibit electrical conductivity, and "ion conduction (conductivity)" means that ions migrate to thereby exhibit electrical conductivity.

In other words, the silver particles form a network of the conductive particles (silver particles) in the silver coating layer 12 by means of their electron conductivity. Furthermore, causing ions to exist among the silver particles allows the ions to migrate in the silver coating layer 12 to thereby exhibit ion conductivity.

Meanwhile, the water absorbent polymer formed of a modified polyalkylene oxide also functions as an electrical conductivity improving material that suitably forms a path for migration of the ions in the silver coating layer 12 to thereby facilitate ion conduction. In other words, not only construction of a network among the silver particles is facilitated but also an effect of facilitating ion conduction is exhibited, by the water absorbent polymer formed of a modified polyalkylene oxide.

As a result, in addition to an electrical conduction mechanism by means of electron conduction formed of the network of the conductive particles, an electrical conduction mechanism by means of ion conduction formed by migration of ions is satisfactorily formed. Electron conductivity and ion conductivity synergistically act to reduce the surface resistance and lead to excellent electrical conductivity. Additionally, even in the case where external force such as bending is applied to completely cut the network of the conductive particles (silver particles), ion conduction complements electrical conduction among conductive particles to suppress an increase in the resistance. For this reason, if the bioelectrode is repetitively deformed, a property with which the electrical conductivity is satisfactorily maintained (strain resistance) is improved.

Fig. 2 is a view conceptually illustrating one example of ion conduction facilitation by a water absorbent polymer formed of a modified polyalkylene oxide, depicting how a polyether chain in the water absorbent polymer coordinates to an ion (here, a cation denoted by (+) in the figure) to stabilize the dissociation state of the ion as well as to form a path through which the ion migrates. The ion can migrate along a polyether chain or from a polyether chain to another polyether chain by the presence of the water absorbent polymer formed of a modified polyalkylene oxide, and thus, ion conduction is facilitated.

The amount of the water absorbent polymer formed of a modified polyalkylene oxide to be added in the silver coating layer 12 is not especially limited, and can be appropriately set in a range where the silicone rubber, which is to be the binder, can be cured. For example, the amount can be set in the range of 2 parts by mass or more and 100 parts by mass or less based on 100 parts by mass of the silicone rubber.

In the present invention, the silver coating layer 12 is preferably caused to further contain a modified silicone. This enables the effects of the present invention to be more satisfactorily exhibited.

As the modified silicone, ones obtained by introducing a side chain that causes modification to the main chain formed of a siloxane bond (-Si-O-; also referred to as a silicone chain) can be preferably used. Examples thereof include silicones containing polyether modification, polyether-alkyl comodification, polyglycerin modification, polyglycerin-alkyl comodification, or the like. The side chain that causes modification preferably contains an ether bond (-C-O-C-).

As the polyether-modified silicone, ones obtained by introducing a side chain formed of a polyether chain into the main chain formed of a silicone chain can be used.

As the polyether-alkyl-comodified silicone, ones obtained by introducing a side chain formed of a polyether chain and a side chain formed of an alkyl chain into the main chain formed of a silicone chain can be used.

As the polyglycerin-modified silicone, ones obtained by introducing a side chain formed of a polyglycerin chain into the main chain formed of a silicone chain can be used.

As the polyglycerin-alkyl-comodified silicone, ones obtained by introducing a side chain formed of a polyglycerin chain and a side chain formed of an alkyl chain into the main chain formed of a silicone chain can be used.

Among these, the polyether-modified silicone or polyglycerin-modified silicone is particularly preferable.

The modified silicone preferably has a viscosity of 100 mm²/s or more and 5000 mm²/s or less and preferably has an HLB of about 1 or more and 15 or less.

One of the modified silicones may be used singly or a plurality of the modified silicones may be used in combination. The modified silicone facilitates dispersion of the silver particles into the binder (silicone rubber) and further facilitates construction of a network of the conductive particles (silver particles). Furthermore, when the modified silicone can coordinate to ions, the modified silicone cooperates with the water absorbent polymer formed of a modified polyalkylene oxide mentioned above to suitably form a path for migration of the ions in the silver coating layer 12 and further facilitate ion conduction.

In the case where the silver coating layer 12 is caused to contain the modified silicone, the amount thereof to be added is not especially limited and can be appropriately set in a range where the silicone rubber, which is to be the binder, can be cured. The amount can be set in the range of 2 parts by mass or more and 100 parts by mass or less based on 100 parts by mass of the silicone rubber.

The silver particles are not especially limited as long as the silver particles are dispersible in the silicone rubber. For example, at least one type of aggregated silver powders and flaky silver powders can be used. An aggregated silver powder and a flaky silver powder may be mixed and used, or either one type of them may be used.

The aggregated silver powder refers to a silver powder of a plurality of particulate primary particles three-dimensionally aggregated, and an example thereof can be trade name: "G-35" (manufactured by DOWA Electronics Materials Co., Ltd.).

The flaky silver powder refers to a silver powder having a scale-like shape, and examples thereof can include trade name: "327077" (manufactured by Sigma-Aldrich Co. LLC.) and trade name: "FA-D-3" (manufactured by DOWA Electronics Materials Co., Ltd.).

The average particle size of the silver particles is not especially limited, but is preferably in the range of 4 µm or more and 8 µm or less in the case of aggregated ones and preferably in the range of 5 µm or more and 15 µm or less in the case of flaky ones. The average particle size is an average diameter determined by measurement of an electron micrograph and calculation using arithmetic mean.

The total amount of the silver particles to be compounded in the silver coating layer 12 can be appropriately set in the range where electrical conductivity can be imparted, but is preferably in the range of 50 parts by mass or more and 600 parts by mass or less, more preferably in the range of 100 parts by mass or more and 400 parts by mass or less, based on 100 parts by mass of the silicone rubber.

The film thickness of the silver coating layer 12 is not especially limited and is preferably in the range of 10 µm or more and 300 µm or less, more preferably in the range of 15 µm or more and 100 µm or less. This can further enhance adhesion of the silver coating layer 12 with respect to the conductive rubber electrode 11, further prevent delamination of the silver coating layer 12, and additionally lower the contact impedance.

In the case of the conductive rubber electrode 11 mentioned above is in the form of sheet, the film thickness of the silver coating layer 12 can be made smaller than the film thickness of the conductive rubber electrode 1.

In the silver coating layer 12, the type of ions caused to be present among the silver particles is not especially limited, but from the viewpoint of imparting satisfactory ion conductivity, ions derived from a salt such as an inorganic salt (ions obtained by electric dissociation of a salt) are preferable.

Examples of the inorganic salt include chloride salts, sulfates, and carbonates.

Examples of the chloride salt include sodium chloride, potassium chloride, lithium chloride, calcium chloride, and magnesium chloride.

Examples of the sulfate include sodium sulfate, potassium sulfate, lithium sulfate, calcium sulfate, and magnesium sulfate.

Examples of the carbonate include sodium carbonate, potassium carbonate, lithium carbonate, calcium carbonate, and magnesium carbonate.

Among these, from the viewpoint of ion mobility or the viewpoint of solubility in a liquid such as water used for the salt water treatment mentioned below and the like, preferable are chloride salts of an alkali metal such as sodium chloride, potassium chloride, and lithium chloride.

For example, as shown in Fig. 3, by use of the bioelectrode 1 according to the present embodiment, the conductive rubber electrode 11 is connected to a measuring apparatus via a signal transmission member 14 such as wiring, the surface of the silver coating layer 12 is caused to contact a living body 3, and electrical signals from the living body 13 can be measured in the measuring apparatus.

Use of the bioelectrode 1 according to the present embodiment for measuring an electrocardiogram as electrical signals is particularly preferable. The bioelectrode 1 according to the present embodiment can be suitably used in medical measuring apparatuses, wearable measuring apparatuses, and health monitoring devices, for example.

In one example of a method for producing the bioelectrode 1 according to the present embodiment, first, a conductive rubber electrode 11 is provided, and a silver coating layer 12 is formed on the conductive rubber electrode.

On forming the silver coating layer 12, first, silver particles, a water absorbent polymer formed of a modified polyalkylene oxide, and further preferably a modified silicone are mixed to an uncured liquid silicone rubber (binder), and the mixture is stirred to prepare a silver paste. Meanwhile, it is possible to appropriately compound a crosslinking agent for crosslinking (curing) the silicone rubber to the silver paste. Thereafter, the silver paste prepared is applied on the conductive rubber electrode 11. Curing the silver paste applied by heating causes the silver coating layer 12 to be formed.

On curing the silver paste (i.e., on crosslinking the silicone rubber), it is preferable to heat the silver paste to a temperature equal to or higher than the melting point of the water absorbent polymer formed of a modified polyalkylene oxide. Thereby, the water absorbent polymer formed of a modified polyalkylene oxide melts and exhibits high affinity to both the silver particles and the binder (silicone rubber). Then, the silver particles and the binder are allowed to be mixed well to enable construction of a network among the silver particles to be facilitated.

In the case where ions of an inorganic salt are caused to exist in the silver coating layer 12, the method therefor is not especially limited, but, for example, it is possible to use a method including mixing an inorganic salt in an uncured paste for forming the silver coating layer 12.

An inorganic salt has a low solubility in the paste and is unlikely to be ionized in the paste. Usually, even when such an inorganic salt is mixed in the paste, it is difficult to cause the inorganic salt to exist as ions in the silver coating layer 12 to be obtained. In contrast to this, as long as the modified polyalkylene oxide or the modified silicone is contained in the paste, dissociation of the inorganic salt to ions is facilitated, and thus, it is possible to cause the inorganic salt to exist as ions in the silver coating layer 12 to be obtained.

Alternatively, instead of adding the inorganic salt directly to the paste, it is also possible to, for example, dissolve (ionize) the inorganic salt in a solvent such as water to prepare a solution and add this solution to the paste. In the method of adding the solution to the paste, there is a limit on the solubility (compatibility) between the solvent such as water and the paste. In the case where the compatibility is low, the aqueous solution and the paste separate, and the inorganic salt is likely to be unevenly distributed in the silver coating layer 12 to be obtained. Then, the following method can be further preferably used.

In other words, as a further preferable method of causing the ions of the inorganic salt to exist in the silver coating layer 12, it is possible to use a method of immersing a silver coating layer 12 (to which no inorganic salt has not been added yet) formed by drying and curing the paste in a solution of an inorganic salt (also referred to as a salt water treatment). This causes the ions of the inorganic salt dissociated in the solution to penetrate the silver coating layer 12 to thereby enable the ions of the inorganic salt to suitably exist in the silver coating layer.

Particularly, molecular chains of a silicone rubber have high mobility, and thus gas or liquid molecules penetrate in the molecular chains more easily, by dissolution or diffusion, than in resins, which are also polymers. It is possible to cause the ions in the inorganic salt in the solution to penetrate from the surface of the silver coating layer by use of this property. This penetration behavior is facilitated by the combination of the silicone rubber and the water absorbent polymer formed of a modified polyalkylene oxide. When a modified silicone is combined, the penetration behavior is further facilitated.

The solvent for use in the solution may be any solvent as long as the solvent can dissolve the inorganic salt, and examples thereof include water, ketones such as acetone and alcohols such as ethanol. Among these, in respect of safety and costs, water, ethanol, or a mixture of water and ethanol is preferable, and water is most preferable.

As described above, an effect of reducing the surface resistance of the bioelectrode can be provided by causing the silver coating layer 12 to contain the water absorbent polymer formed of a modified polyalkylene oxide. Particularly when the silver coating layer 12 has ion conductivity, an effect of enhancing the strain resistance can be provided. This improves the accuracy of the bioelectrode to measure bioelectric signals. For example, even when external force such as bend associated with laundry is applied to remove contact among silver particles, the ion conductivity is maintained to thereby allow the electrical conductivity to be maintained. Additionally, the bioelectrode, which is derived from a silicone rubber, has excellent flexibility. Thus, even when fitted for a long period, the bioelectrode causes no discomfort and can suitably conform to movements of a living body while maintaining satisfactory electrical conductivity.

In the description hereinabove, the case where the bioelectrode 1 is in the form of sheet has been principally described. However, the shape of the bioelectrode is not limited thereto, and the bioelectrode can have various shapes. Meanwhile, the electrode surface part to be contacted with a living body can be constituted by the silver coating layer 12 mentioned above.

### Examples

Hereinafter, the present invention will be described more in detail based on examples conducted to clarify the effects of the present invention. Note that the present invention is not limited by the following examples and comparative examples in any way.

### 1. Production of Bioelectrode

### (Example 1)

### (1) Production of Conductive Rubber Electrode

To 100 parts by mass of a conductive silicone rubber (trade name: "KE-3801M-U"; containing carbon black, manufactured by Shin-Etsu Chemical Co., Ltd.), 1.0 parts by mass of a crosslinking agent (trade name "C-8A"; 2,5-dimethyl-2,5-bis(t-butylperoxy)hexane content: 80% by mass, manufactured by Shin-Etsu Chemical Co., Ltd.) was compounded.

Thereafter, a material obtained by kneading the components compounded described above in a kneader for 10 minutes and then further kneading the components with a roll for three minutes, (carbon black content: 6% by volume) was press-crosslinked (primary-crosslinked) at 180°C for four minutes and thereafter secondary-crosslinked at 230°C for five hours to provide a conductive rubber electrode having a thickness of 1 mm.

### (2) Formation of Silver Coating Layer

To 100 parts by mass of a liquid silicone rubber (trade name: "KE-106", manufactured by Shin-Etsu Chemical Co., Ltd.) as a binder, 150 parts by mass of each of two types of silver particles (trade name: "FA-D-3" and trade name: "G-35"; both manufactured by DOWA Electronics Materials Co., Ltd.) (total amount to be compounded: 300 parts by mass), additionally, as electrical conductivity improving materials, 10 parts by mass of a polyether-modified silicone (trade name: "KF-6015", manufactured by Shin-Etsu Chemical Co., Ltd.) and 10 parts by mass of a polyglycerin-modified silicone (trade name: "KF-6106", manufactured by Shin-Etsu Chemical Co., Ltd.), and 10 parts by mass of a water absorbent polymer formed of a modified polyalkylene oxide (trade name: "AQUA CALK TWB"; pulverized product, melting point: 60 to 65°C, manufactured by Sumitomo Seika Chemicals Company, Limited.) were mixed, and the mixture was stirred to prepare a silver paste.

Thereafter, the silver paste prepared was applied by screen printing on the conductive rubber electrode obtained in the above-described "(1) Production of Conductive Rubber Electrode" and cured in an oven set at 150°C for 30 minutes to thereby form a silver coating layer having a film thickness of 54 µm.

Thereafter, as a salt water treatment, the silver coating layer 12 was immersed in a sodium chloride aqueous solution having a concentration of 1% for an hour and then dried. As mentioned above, a bioelectrode was produced.

### (Example 2)

A bioelectrode was produced in the same manner as in Example 1 except that compounding of the modified silicone was omitted.

### (Example 3)

A bioelectrode was produced in the same manner as in Example 1 except that 20 parts by mass of a polyether-modified silicone (trade name: "KF-6015", manufactured by Shin-Etsu Chemical Co., Ltd.) was singly used as the modified silicone.

### (Example 4)

A bioelectrode was produced in the same manner as in Example 1 except that 20 parts by mass of the polyglycerin-modified silicone (trade name: "KF-6106", manufactured by Shin-Etsu Chemical Co., Ltd.) was singly used as the modified silicone.

### (Reference Example 1)

A bioelectrode was produced in the same manner as in Example 1 except compounding of the water absorbent polymer formed of a modified polyalkylene oxide was omitted.

### (Comparative Example 1)

A bioelectrode was produced in the same manner as in Reference Example 1 except that compounding of the modified silicone was omitted and the film thickness of the silver coating layer was set to 64 µm.

### 2. Evaluation Method

### (1) Electrocardiogram Measurement

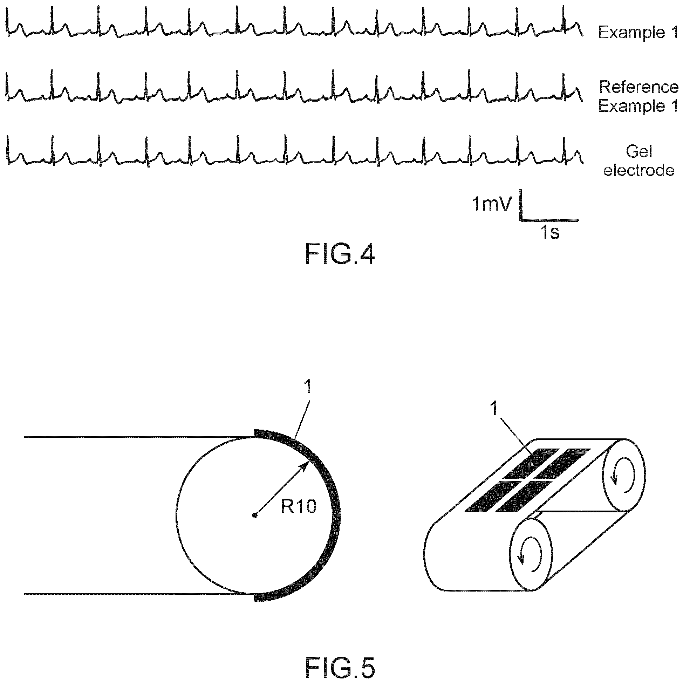

The bioelectrodes (sheets) obtained in Example 1 and Reference Example 1 were each punched to a diameter of 19 mm to produce a bioelectrode for electrocardiogram measurement, and a circuit to be connected to a human body and an electrocardiogram measuring apparatus was formed. Thereafter, an electrocardiogram of an adult male was measured, and waveforms displayed in the electrocardiograph were recorded. Additionally, as a reference, a commercially available gel electrode (wet electrode) was used to record electrocardiogram waveforms in the same manner. The results are shown in Fig. 4.

### (2) Surface Resistance

The silver coating layer surface of each bioelectrode (before the bending test mentioned below) obtained in Examples 1 to 4, Reference Example 1, and Comparative Example 1 was measured for the surface resistance using a low resistivity meter "Loresta" manufactured by Mitsubishi Chemical Analytech Co., Ltd. (using a PSP terminal) by a four-terminal method. The results are shown in Table 1.

### (3) Strain Resistance

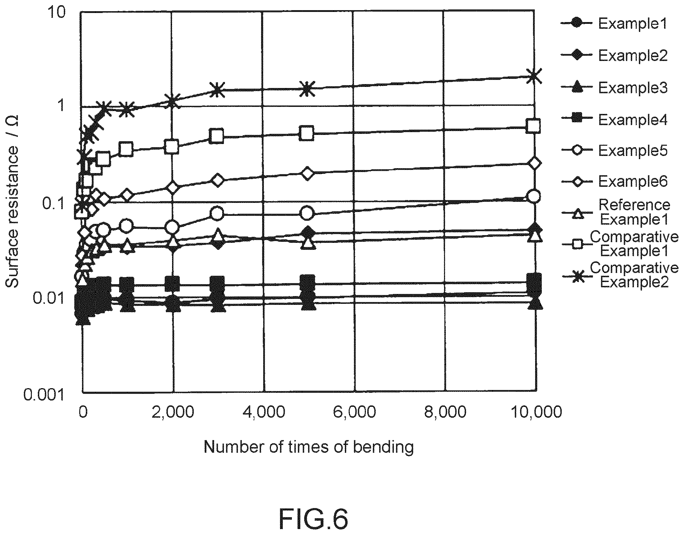

The bioelectrodes obtained in Examples 1 to 4, Reference Example 1, and Comparative Example 1 were each punched to a size of 20 mm × 60 mm. A bending test was conducted in which the surface of the conductive rubber electrode of each bioelectrode was attached onto a conveyor belt illustrated in Fig. 5 and rotated to repetitively apply deformation (external force). Here, each bioelectrode was bent at a radius of 10 mm, and a total of 10000 times of bending was conducted in 5000 seconds (2 times/second). The surface resistance was measured every prescribed number of times in the same manner as in the above-described "(2) Surface Resistance". The results are shown in Fig. 6. Additionally, the measurement results of the change rate of the surface resistance after 10000 times of bending based on the initial surface resistance are shown in Table 1.

**[Table 1]**

| | | | Example 1 | Example 2 | Example 3 | Example 4 | Reference Example 1 | Comparative Example 1 |
|---|---|---|---|---|---|---|---|---|
| Silver coating layer (amount compounded expressed in parts by weight) | Binder | KE-106 | 100 | 100 | 100 | 100 | 100 | 100 |
| | Silver particles | FA-D-3 | 150 | 150 | 150 | 150 | 150 | 150 |
| | | G-35 | 150 | 150 | 150 | 150 | 150 | 150 |
| | Modified silicone | KF-6015 (polyether-modified silicone) | 10 | - | 20 | - | 10 | - |
| | | KF-6106 (polyglycerin-modified silicone) | 10 | - | - | 20 | 10 | - |
| | Water absorbent polymer | AQUA CALK | 10 | 10 | 10 | 10 | - | - |
| | Salt water treatment | | Yes | Yes | Yes | Yes | Yes | Yes |
| Evaluation | Surface resistance [Ω] | | 0.0075 | 0.023 | 0.0064 | 0.0084 | 0.0145 | 0.0328 |
| | Strain resistance (bending test) | Initial surface resistance [Ω] | 0.0068 | 0.0217 | 0.0063 | 0.0089 | 0.0154 | 0.0775 |
| | | Surface resistance after 10000 times of bending [Ω] | 0.0111 | 0.0496 | 0.0086 | 0.0139 | 0.0439 | 0.581 |
| | | Change rate [times] | 1.64 | 2.29 | 1.37 | 1.57 | 2.85 | 7.50 |

### 3. Evaluation

It can be seen from Fig. 4 that the bioelectrodes of Example 1 and Reference Example 1 each can measure an electrocardiogram comparable to that of a commercially available gel electrode (wet electrode) and satisfactorily function as a bioelectrode.

It can be seen from Table 1 that the surface resistance of the bioelectrode of Example 1 has reduced to approximately a half of the surface resistance of Reference Example 1 and to approximately a quarter of the surface resistance of Comparative Example 1, the ion conductivity imparted by the salt water treatment has been facilitated by the water absorbent polymer formed of a modified polyalkylene oxide, and an effect of enhancing the electrical conductivity is considerable.

From Table 1 and Fig. 6, when the bending test was conducted 10000 times, the absolute value of the surface resistance of Example 1 was a quarter of that of Reference Example 1 and approximately one-fiftieth of that of Comparative Example 1. Also as for the change rate in the surface resistance, the change rate of Example 1 is 1.64 times, which stays at approximately a half of that of Reference Example 1 and stays at approximately one-fifth of that of Comparative Example 1, and it can be seen that the strain resistance is enhanced.

Also as for Examples 2 to 4, an excellent effect was confirmed as in Example 1.

### 4. Omission of Salt Water Treatment

### (Example 5)

A bioelectrode was produced in the same manner as in Example 3 except that the salt water treatment was omitted. The results obtained by evaluating the bioelectrode in the same manner as in Example 1 are shown Table 2 and Fig. 6.

### (Example 6)

A bioelectrode was produced in the same manner as in Example 4 except that the salt water treatment was omitted. The results obtained by evaluating the bioelectrode in the same manner as in Example 1 are shown Table 2 and Fig. 6.

### (Comparative Example 2)

A bioelectrode was produced in the same manner as in Comparative Example 1 except that the salt water treatment was omitted. The results obtained by evaluating the bioelectrode in the same manner as in Example 1 are shown Table 2 and Fig. 6.

**[Table 2]**

| | | | Example 5 | Example 6 | Comparative Example 2 |
|---|---|---|---|---|---|
| Silver coating layer (amount compounded expressed in parts by weight) | Binder | KE-106 | 100 | 100 | 100 |
| | Silver particles | FA-D-3 | 150 | 150 | 150 |
| | | G-35 | 150 | 150 | 150 |
| | Modified silicone | KF-6015 (polyether-modified silicone) | 20 | - | - |
| | | KF-6106 (polyglycerin-modified silicone) | - | 20 | - |
| | Water absorbent polymer | AQUA CALK | 10 | 10 | - |
| | Salt water treatment | | No | No | No |
| Evaluation | Surface resistance [Ω] | | 0.0133 | 0.022 | 0.0479 |
| | Strain resistance (bending test) | Initial surface resistance [Ω] | 0.0161 | 0.0273 | 0.0916 |
| | | Surface resistance after 10000 times of bending [Ω] | 0.109 | 0.247 | 2.03 |
| | | Change rate [times] | 6.78 | 9.04 | 22.2 |

From Table 2 and Fig. 6, also in the test examples in which the salt water treatment was omitted, it can be seen that Examples 5 and 6, which contain the water absorbent polymer formed of a modified polyalkylene oxide, have lower surface resistance and more excellent strain resistance in comparison with Comparative Example 2, which contains no water absorbent polymer. It is conceived that the water absorbent polymer formed of a modified polyalkylene oxide allows the silver particles and the binder to be mixed well to thereby facilitate construction of a network among the silver particles.

### List of Reference Signs

- 1: bioelectrode
- 11: conductive rubber electrode
- 12: silver coating layer
- 13: living body
- 14: signal transmission member

## Claims

1. A bioelectrode, **characterized by** comprising:
a conductive rubber electrode containing a rubber and conductive carbon particles; and
a silver coating layer provided on the conductive rubber electrode and containing a silicone rubber and silver particles, wherein
the silver coating layer further contains a water absorbent polymer formed of a modified polyalkylene oxide.

2. The bioelectrode according to claim 1, wherein the silver coating layer includes ions of ion conduction present among the silver particles.

3. The bioelectrode according to claim 2, wherein the ions include at least one selected from the group consisting of chloride salts, sulfates, and carbonates.

4. The bioelectrode according to any one of claim 1 to claim 3, wherein the melting point of the water absorbent polymer is equal to or lower than the crosslinking temperature of the silicone rubber.

5. The bioelectrode according to any one of claim 1 to claim 4, wherein the silver coating layer further contains a modified silicone.

6. The bioelectrode according to claim 5, wherein the modified silicone contains at least one selected from the group consisting of polyether-modified silicones, polyether-alkyl-comodified silicones, polyglycerin-modified silicones, and polyglycerin-alkyl-comodified silicones.
